Europäisches Patentamt

European Patent Office

Office européen des brevets

(19)

(11) Publication number: **0 408 280 A2**

# EUROPEAN PATENT APPLICATION

(12)

(21) Application number: 90307463.1

(22) Date of filing: 09.07.90

(51) Int. Cl.5: **B01L 7/00**

(30) Priority: 08.07.89 GB 8915680

(43) Date of publication of application:
16.01.91 Bulletin 91/03

(84) Designated Contracting States:
**AT BE CH DE DK ES FR GB GR IT LI LU NL SE**

(71) Applicant: **TECHNE (CAMBRIDGE) LIMITED**
**Duxford**
**Cambridge CB2 4PZ(GB)**

(72) Inventor: **Norton, David R.**
**6 Rotterdam Drive, Avon Village**
**Christchurch, Dorset BH23 1HB(GB)**

(74) Representative: **Johnson, Terence Leslie et al**
**Edward Evans & Co. Chancery House 53-64**
**Chancery Laneane**
**London WC2A 1SD(GB)**

(54) Heat resistant multiwell plates.

(57) A novel and improved method of conducting elevated temperature experiments based predominantly on the 96 microwell format utilizing, and maintaining complete compatibility with current multiwell cleaning, dispensing and reading machinery. This includes a specifically designed heat programmable block, whereby the novel 96 microwell plates (10, 11), fit into such an aluminium heat programmable block so that various heating cycles can be additionally performed on say, small volumes of DNA solutions placed in the individual microwells, the multiwell plates (10, 11) being formed of polycarbonate and dimensionally stable up to about 125°C.

Fig. (2a).

Fig. (2b).

## HEAT RESISTANT MULTIWELL PLATES

This invention relates to the technology of multiwell plates of the type that are extensively used in serology, virology immunology and various other scientific disiplines that commonly use microtitration methods for numerous diagnostic test applications such as enzyme linked chemical assays.

At the present time the customary format for such microwell plates is an injection moulded Polystyrene plate measuring approximately 125 mm by 85 mm by 15 mm moulded into which is a regular array of 96 cups or microwells, arranged in twelve columns and eight rows. The individual microwells are most usually around 10 mm deep with a diameter around 8 mm. Depending upon microwell profile, whereby the individual microwell bases can be flat bottomed ('F'), round bottomed ('U') or cone shaped ('V'), the capacity of each microwell is approximately 300 μL. The centres of each well are spaced 9 mm apart.

It is also necessary that the plates are relatively optically transparent in order that results information concerning the contents of the microwells can be deduced by photometry subsequent to each set of experiments. Most usually this takes the form of a colour density measurement giving a +ve or -ve result.

The universal configuration for multiwell plates, that of 96 microwells, has given rise to the subsequent standardization of all subsidiary and peripheral equipment used to help perform the various test procedures. The equipment and machines capable of handling the 96 microwell format are now established articles of commerce. These include microplate washers, microplate sample dispensers and microplate photometric reading devices.

Consequently, the three afformentioned varieties of laboratory machine have, in particular, help to automate arid speed up all routine diagnostic tests and experimental procedures.

In addition to injection moulded PolyStyrene (PS) multiwell plates there are also available thermoformed PolyVinlyChloride (PVC) and Glycol modified PolyEthyleneTerephalate (PETG). Whereas the PS plates are rigid, the PVC and PETG plates display a degree of flexibility. This is due primarily to their different mode of manufacture.

The configuration of the 'flexible' plates is similar to the 'rigid' plates in that they possess 96 regularly spaced microwells that have 'F', 'V' or 'U' shaped bases when viewed in sectioned profile.

Further, a relatively new branch of molecular biology relates to the study of Deoxyribonucleic Acid (DNA), which is the single most important molecule in living cells and contains all the information that specifies the cell.

Additionally, a number of research and analytical techniques that apply to DNA studies, such as DNA replication and enhancement, DNA sequencing and the generalized topic of polymer chain reactions (PCR) using high temperature polymerases, require as a prerequisite, the use of elevated temperatures, i.e. above around 90°C..

This corresponds to the melting temperature of DNA molecules, Fig.(1)., whereby the DNA molecule which consists of a double stranded helix, seperates. This is illustrated in Fig.(1)., where the state of the DNA molecule in the different regions of the melting curve is shown, whereby the vertical axis depicts a calibrated value for the absorbance of incident light of a particular wavelength on a DNA solution. This is taken as an indication of the conformation of the DNA molecule.

Most experiments require the DNA to initially be separated into the single stranded random coil state, i.e. to experience temperatures in excess of 90 to 95°C.. Certain, other techniques, further require that the DNA is 'annealed', that is, to be held a particular temperatures, around 20 to 25°C below the melting temperature, for extended periods. It is also often the case that a solution of DNA will require the application of numerous temperature cycles between the melting temperature and the annealing temperature, i.e. experiencing 60 to 95°C, and beyond, for time periods in excess of one hour.

It is the object of the current invention to assist in providing a novel and improved method of conducting such elevated temperature experiments based predominantly on the 96 microwell format utilizing, and maintaining complete compatibility with all the current multiwell cleaning, dispensing and reading machinery. This includes a specifically designed heat programmable block, whereby the novel 96 multiwell plates, as described below fit into such an aluminium heat programmable block so that various heating cycles can be additionally performed on say, small volumes of DNA solutions placed in the individual microwells.

Multiwell plates of the types currently available today, formed from PS, PVC and PETG, cannot be utilized for such elevated temperature experiments using such heat blocks because of their lack of temperature stability. At prolonged (a few minutes) temperatures above 75°C all multiwell plates formed from such materials soften and distort rendering them inappropriate for such uses.

The current invention relates specifically to Polycarbonate multiwell plate thermoformings in various formats and sectioned profiles, that enable the following:

Complete compatibility of use with all current multiwell plate processing laboratory equipment, including dispensing, washing and photometric reading machines.

Use at elevated temperatures, up to 125° C if necessary, in association with a heat programmable block, oven or similar device.

Enhanced optical properties with improved optical distortion, gloss and clarity characteristics.

The ability to withstand sterilization by auto claving if desired, with no detrimental effects to shape or optical clarity.

Novel sectioned profiles of the individual microwells, including 'shoulder' features that enable the separate placement of two reactive samples in the same microwell so that many such samples contained within a multiwell plate may be reacted simultaneously. This is particularly important with regard to time sensitive and reaction kinetic experiments.

The invention will now be described by way of example, with reference to the accompanying diagrams, in which;

Fig.(1)., shows a melting curve of DNA illustrating melting temperature, $T_m$ , and possible molecular configurations for various degrees of melting. Relative absorbance values are for incident light at 260 nanometres.

Fig.(2a)., illustrates a plan perspective of a typical polycarbonate multiwell plate with 96 microwells in the standard 12 by 8 format.

Fig.(2b)., gives an individual microwell cross section, taken at X-X', in Fig.(2a)..

Fig.(3a)., illustrates another type of multiwell plate in plan perspective.

Fig.(3b)., again gives a typical cross sectional view of Fig.(3a)., taken at Y-Y'.

Fig.(4a)., is a plan view perspective of a single microwell 'strip' in a configuration of 8 microwells.

Fig.(4b)., is the cross section profile corresponding to Z-Z' in Fig.(4a)..

Fig.(5a)., again is a plan perspective; with the microwells in a configuration of 8 by 2 rows.

Fig.(5b)., is a cross section view of Fig.(5a)., along A-A'.

Figs.(2a)., (3a)., (4a). and (5a) gives a schematic representation of typical plan perspectives of the current Polycarbonate multiwell plate invention, whilst Figs.(2b)., (3b)., (4b). and (5b). illustrate typical cross section profiles of the individual microwells that are formed within each different illustrated Polycarbonate multiwell design.

All multiwell plates, 10, and multiwell strips, 11, consist of a regularly spaced array of small microwells, 12, thermoformed from specific grades of polycarbonate sheeting, 13, approximately 0.6 mm in thickness; although manufacture from other thicknesses is, of course, possible.

In order to maintain compatibility with current laboratory multiwell plate processing equipment the microwells, 12, are always configured so that their centres have a 9.0 mm separation.

The multiwell plates, 10, Figs.(2a). and (3a)., are most usually manufactured in a 96 well format, more specifically, twelve columns and eight rows of microwells, 12. It is further apparent to anyone skilled in the art that that the plates; 10, and the strips, 11, can be fabricated into many different multiwell formats.

As an example, in Figs.(4a). and (5a)., multiwell strips 11, consisting of one row of eight microwells, 12, and two rows of eight microwells, 12, have been illustrated, respectively.

In this way, and using a simple plastic frame. multiwell strips, 11, can bc assembled into multiples of eight or sixteen microwells, 12 , up to a total of 96 microwells.

Further, each format of microwells, 12 , can have different cross sectional profiles, each specifically designed for a particular set of experimental procedures.

In Fig.(2b)., for example, the microwells, 12, have 'U'-shaped bases, 14, and incorporate a shoulder feature, 15. This permits two small solution samples to be placed into the same microwell. More specifically, a large volume is dispensed into the 'U'-shaped base, 14, while a smaller volume is dispensed onto the shoulder feature, 15, to permit the placement with separation of, say, two dissimilar and reactive solution samples within the same microwell.

As previously disclosed the microwell centres are always regularly spaced at 9.0 mm; this obviously constrains maximum microwell diameter to something approaching this value. Additionally, the technique of thermoforming requires a slight taper to the microwell sides, 16, in Fig.(3b). and Fig.(5b).. Also, the maximum microwell depth is constrained by the outside microplate dimensions, most usually, this approaches 13.0 mm, as most microplates are this deep.

In this way, that is, by variation of microwell diameter, microwell depth ( effectively the diameter/depth ratio ) and individual microwell cross section profile, a whole range of different microplates can be fabricated, each specifically designed with regard shape and capacity to optimize their performance for a particular application and sample solution volume.

The potential of variations possible are further depicted in Fig. (3b):, where the microwells, 12, have a depth > diameter, in association with a small sample volume and flat microwell base, 14.

Fig.(4b). indicates yet another example of the current invention; this time however, the microwell strip, 11, of Fig.(4a)., that has one row of eight microwells, has microwells, 12, that possess 'V'-shaped bases, 14, and a shoulder feature, 15, when viewed in cross section, for the provision of a separate solution samples.

Fig.(4b). indicates yet another example of the current invention; this time however, the microwell strip, 11, of Fig.(4a)., that has one row of eight microwells, 12, that possess 'V'-shaped bases, 14, and a shoulder feature, 15, when viewed in cross section, for the provision of a separate solution samples.

Finally, Fig.(5). illustates one further example of the current Polycarbonate multiplate and multistrip invention, whereby the microwell strip, 11, of Fig.-(5a)., that is configured into a format of two eight microwell rows, have microwells that possess flat bases, 14.

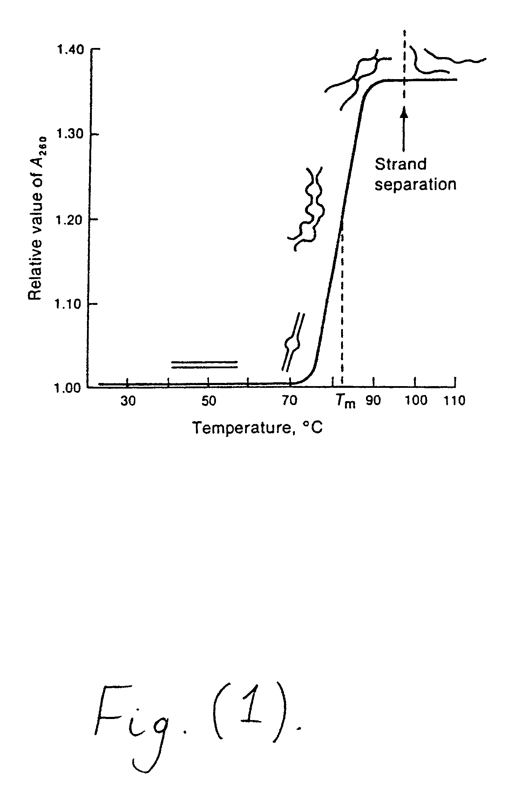

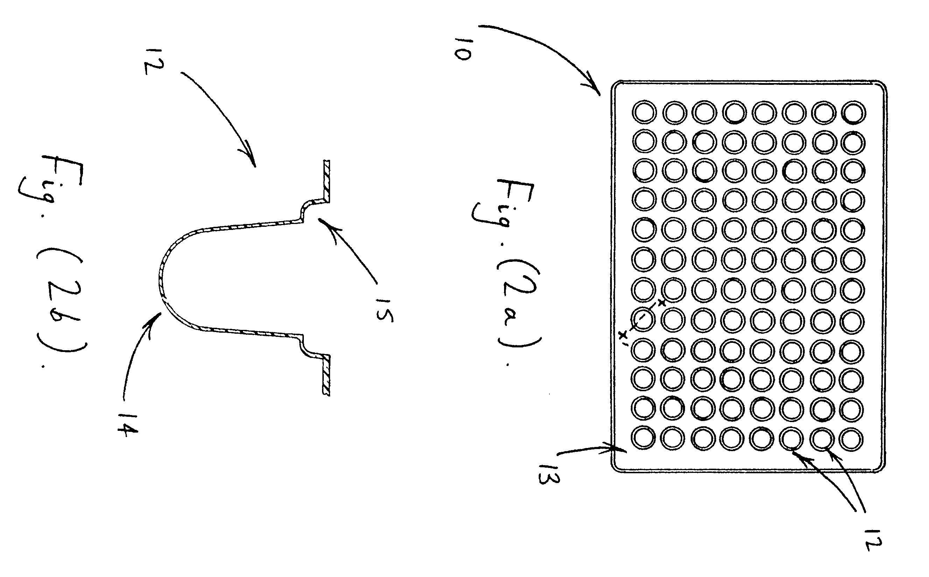

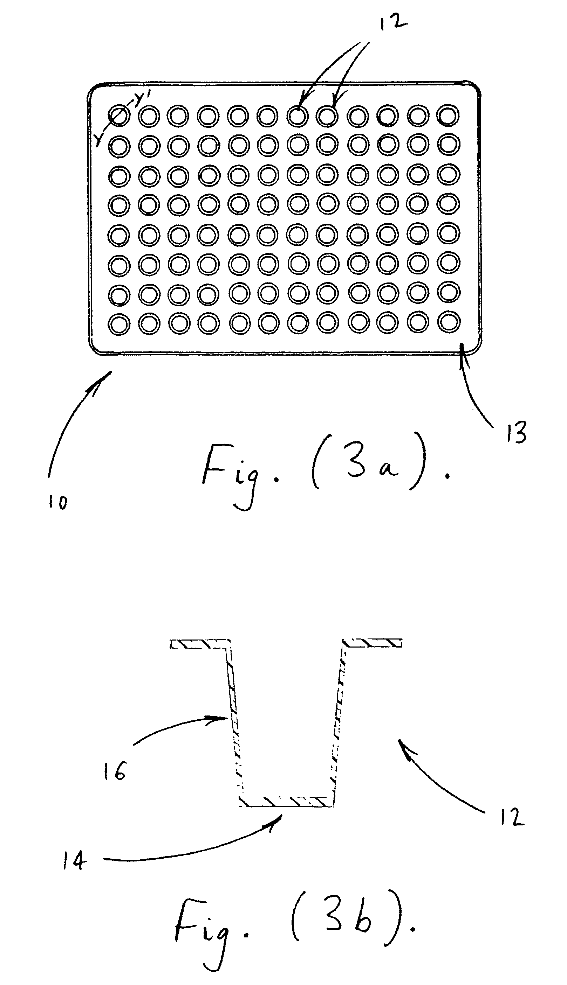

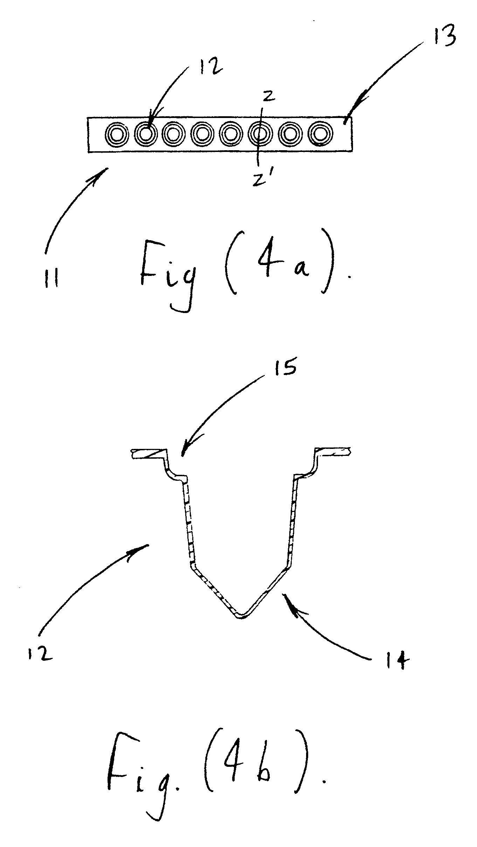

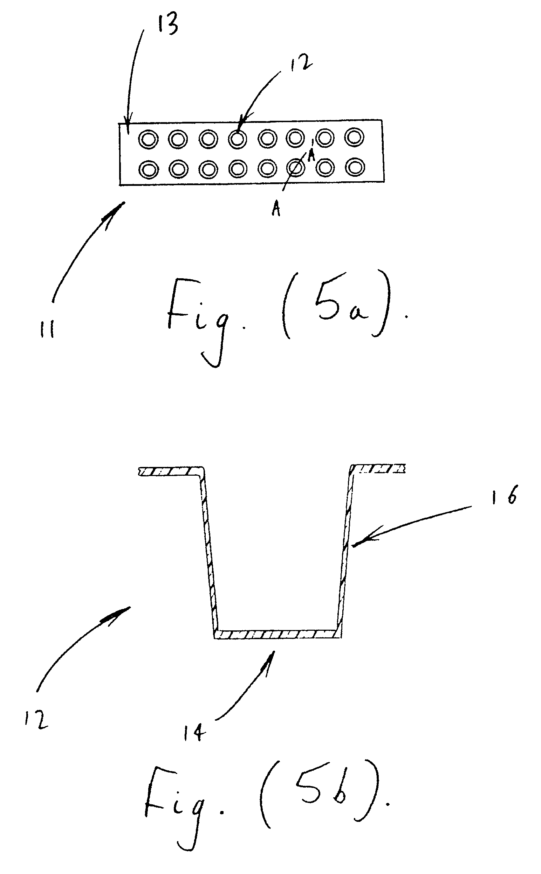

## Claims

1. A plate for use in a heat treatment process, integrally formed from a mouldable material and having a plurality of spaced wells for receiving material to be treated, characterised by the plate (10, 11) comprising a thermoformed material which is dimensionally stable at temperatures up to about 125°C.

2. A plate according to Claim 1, characterised in that the material is a polycarbonate material.

3. A plate according to Claim 1 or Claim 2, characterised in that each well (12) has a depth greater than its diameter.

4. A plate according to any preceding Claim, characterised in that each well (12) has a shoulder and an additional well configuration (15) adjacent each shoulder whereby two different materials may be treated in each well (12, 15).

5. A plate according to Claim 4, characterised in that the base (14) of each well (12) is of substantially V-shape.

6. A plate according to Claim 4, characterised in that the base (14) of each well (12) is of outwardly convex configuration.

7. A plate according to any preceding Claim, characterised in that the wells (12) are arranged as a strip (11).

8. A heat treatment system, characterised in that a heat programmable heating device having means to accommodate a plate (10, 11) according to Claim 1 is mounted in said means.

9. A heat treatment system according to Claim 8, characterised in that the heating device comprises a heating block comprising a plurality of orifices adapted to provide said means for receiving the wells (12) of the plate (10, 11).

10. A heat treatment system according to Claim 9, characterized in that the heating device comprises an aluminium block comprising a plurality of orifices adapted to provide said means for receiving the wells (12) of the plate (10, 11).

Fig. (1).

Fig.(2a).

Fig. (2b).

Fig. (3a).

Fig. (3b).

Fig (4a).

Fig. (4b).

Fig. (5a).

Fig. (5b).